# EUROPEAN PATENT SPECIFICATION

(11) **EP 1 502 609 B1**
(45) Date of publication and mention of the grant of the patent: **26.08.2015**
(21) Application number: 04017610.9
(22) Date of filing: 26.07.2004
(51) Int. Cl.: A61M 25/10, A61L 29/12

(54) **Catheter with expandable member**
Katheter mit ausziehbarem Element
Cathéter avec un élément expansible

(30) Priority: 29.07.2003 JP 2003202958
(43) Date of publication of application: 02.02.2005
(73) Proprietor: Terumo Kabushiki Kaisha, Tokyo (JP)
(72) Inventor: Suzuki, Yorito, Fujinomiya-shi Shizuoka (JP); Kitada, Noriyuki, Fujinomiya-shi Shizuoka (JP); Kumoyama, Kenichi, Fujinomiya-shi Shizuoka (JP)
(74) Representative: Casalonga, Axel

(56) References cited:
- EP-A- 1 192 970
- EP-A- 1 388 346
- WO-A-01/34062
- WO-A-99/32184
- WO-A1-96/03162
- WO-A1-96/03163
- US-A- 5 868 704
- US-A1- 2002 081 404
- US-A1- 2003 065 355
- US-B1- 6 303 697
- SUBRAMONEY S: "NOVEL NANOCARBONS- STRUCTURE, PROPERTIES, AND POTENTIAL APPLICATIONS" ADVANCED MATERIALS, VCH VERLAGSGESELLSCHAFT, WEINHEIM, DE, vol. 10, no. 15, 20 October 1998 (1998-10-20), pages 1157-1171, XP000783181 ISSN: 0935-9648

## Description

The present invention relates to a catheter with expandable member which is used as a living organ expanding appliance for a procedure of dilating a constricted part (stenosis part) or occluded part formed in a living organ and in particular the present invention relates to a catheter with exapandable member according to the preamble of claim 1, such as it is for example known from US2003/065355A1, WO96/03162A1, EP 1 388 346 A.

As a treatment of constriction or occlusion of a living lumen or body cavity such as blood vessels, bile duct, esophagus, trachea, urethra or the like, there has been practiced a living organ expansion method for introducing an expandable member such as a balloon and a stent into the diseased part (constricted part or occluded part). For example, a procedure in which a balloon attached to a portion near the distal end of an elongated tubular member (catheter) is inserted to the diseased part (constricted part or occluded part) and a pressure fluid is introduced into the balloon to expand the balloon or a procedure of securing a lumen or body cavity space by setting a stent to indwell therein has been practiced. The stent includes those of the type in which the stent itself has an expanding function (self-expandable stent), and those of the type in which the stent is mounted on a balloon and is expanded (plastically deformed) by the expanding force of the balloon to be brought into close contact with and fixed to the inside surface of a target site (balloon-expandable stent).

The above-mentioned expandable member is guided to the target site by a guide wire passed through the catheter one end of which is attached to a distal end portion of the expandable member. In this case, in order to ensure that pushing forces exerted from the outside of a living body are effectively transmitted to the distal end portion of the catheter without energy loss and the catheter can pass through bent portions and occluded portions of blood vessels to reach the target site as sliding over the guide wire with good operation property, it is important to reduce the frictional (sliding) resistance of the guide wire, and, therefore, it is desirable that the inside surface of the catheter (tubular member) has a low-friction property and a high slidability. In the conventional catheters with expandable member, it is supposed that the slidability between the guide wire and the tubular member wherein the guide wire passes through is not sufficient, and, therefore, it is desired to improve their slidability. In addition, the tubular member is desired to have such flexibility as to permit operations corresponding to bent portions of blood vessels. Furthermore, the portion, for attachment of the expandable member, of the tubular member is required to have substantial flexibility, since kinking and a lowering in operability would be generated if the portion is too hard as compared to the portions on the distal and proximal sides thereof.

As a material for the low-friction property mentioned above, there is known polyolefins. However, polyolefins are poor in adhesion property and in the property for heat-fusing to various materials, and tend to be higher in hardness and poorer in flexibility as they have lower-friction properties. In view of this problem, there has been disclosed a configuration in which, for example, the inside surface layer of a tubular member for passing a guide wire therethrough of an expansion catheter is formed of a polyolefin and the outside surface layer for attachment of an expandable member thereon of the tubular member is formed of a thermoplastic elastomer or the like (see, for example, Japanese Patent Laid-open No. Hei 11-151292, EP 1192970 A1).

WO 99/32184 discloses a balloon catheter system in which the outer surface of the balloon may have a coating of fullerenes which are preferably photo-sensitive or activated by light. When activated by light, the fullerene coating may give off therapeutic oxygen radicals intended to affect local cell function to prevent or reduce cell proliferation, such as to limit restenosis after angioplasty.

An article by Subramoney entitled, "Novel Nanocarbons - Structure, properties and potential applications" in Advanced Materials (1998), Volume 10. No. 15, pages 1157-1171, discloses various uses, trends and potential applications for carbon nanostructures.

The present invention provides a catheter with expandable member in which a tubular member for passing a guide wire therethrough has an inside surface of its lumen having a low-friction property and a high slidability, the guide wire is low in sliding resistance, which is excellent in flexibility of the tubular member and in flexibility of an attachment portion between an expandable member and the tubular member, which can be inserted into a bend portion of a blood vessel without kinking, and which is excellent in operation property and safety. The present invention provides a catheter with expandable member according to claim 1. The dependent claims relate to advantageous embodiments.

The catheter with expandable member according to the present invention is suitable for use as a living organ expanding appliance for the expansion or the dilatation of a constricted portion (stenosis portion) or occluded portion formed in a living body organ such as blood vessels, bile duct, trachea, esophagus, urethra and other organs. Particularly, the catheter with expandable member according to the invention is excellent in flexibility, is low in sliding resistance of the guide wire, and ensures that introduction of an expandable member such as a balloon to a constricted portion of a cardinal blood vessel or the like or introduction of a stent mounted on the expandable member such as a balloon can be carried out with safe and good operation property.

The above and other objects, features and advantages of the present invention will become apparent from the following description and appended claims, taken in conjunction with the accompanying drawings, in which:
Fig. 1 is a front view showing one embodiment of the catheter with expandable member according to the present invention;
Fig. 2 is an enlarged sectional view of an expandable member and the vicinity thereof in the catheter with expandable member of Fig. 1;
Fig. 3 is an enlarged sectional view of a portion near the distal end of a catheter with expandable member, showing another embodiment of the present invention;
Fig. 4 is a sectional view taken along line III-III of Fig. 3;
Fig. 5 is an illustration of a three-point bending test in Examples; and
Fig. 6 is an illustration of a slidability test of the guide wire in Examples.

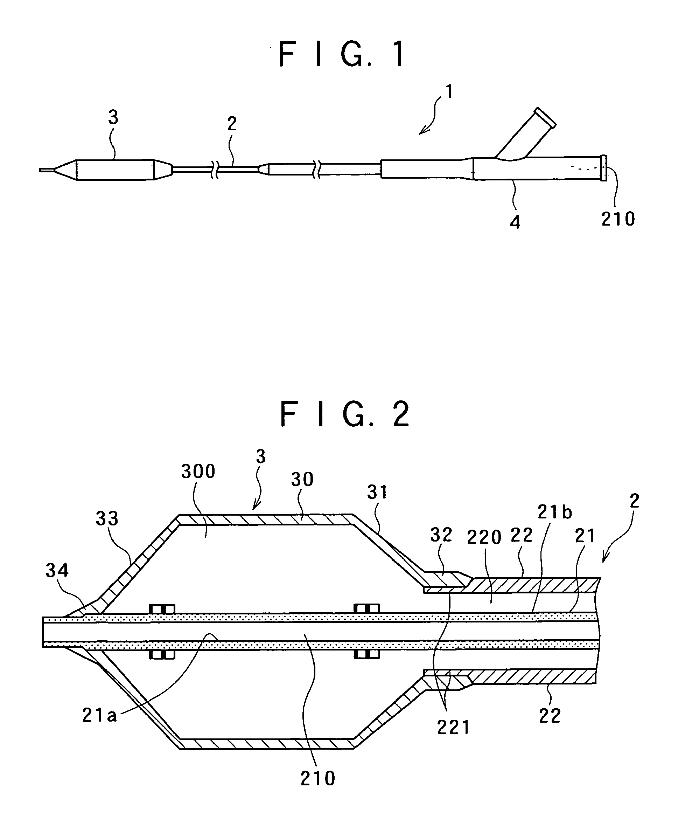

The catheter with expandable member (living organ expanding appliance) according to the present invention will be described referring to the drawings. In the following Figs. 1 to 3, the right side will be referred to as the "proximal end" side, and the left side as the "distal end" side.

Fig. 1 is a front view of one embodiment of the catheter with expandable member. For illustration of the catheter with expandable member according to the present invention in whole, a balloon catheter for use as a PTCA (percutaneous transluminal coronary angioplasty) catheter or PTA (percutaneous transluminal angioplasty) catheter for expanding or dilating a constricted portion (stenosis portion) or a occluded part of a blood vessel will be taken as an example in the following description, but the invention is not limited to the embodiment shown in Figures. For example, the catheter with expandable member according to the present invention may be a catheter for use in expanding a living lumen or body cavity other than a blood vessel, and may be a catheter comprising a stent mounted on a balloon.

In Fig. 1, the catheter with expandable member 1 comprises a shaft main body portion 2, and an expandable member (for stent expansion) such as a balloon 3 disposed at a distal end portion of the shaft main body portion 2. The catheter with expandable member (balloon) 1 shown in Fig. 1 is of the over-the-wire type in which a lumen for a guide wire 210 is opened at a hub 4 attached to a proximal end portion of the shaft main body portion 2, but the catheter with expandable member according to the present invention is not limited to this type. The catheter with expandable member of the invention may be of any type, for example, the rapid exchange type in which the lumen for a guide wire 210 is opened at an intermediate portion of a shaft main body portion.

Fig. 2 is an enlarged sectional view of an expandable member 3 and the vicinity thereof in the catheter with expandable member 1 shown in Fig. 1.

The shaft main body portion 2 is a concentric double wall tube composed of a guide wire guiding tubular member (hereinafter referred to as inner tube) 21 and a balloon expanding tubular member (hereinafter referred to as outer tube) 22, and the distal end of the outer tube 22 located slightly on the proximal side relative to the distal end of the inner tube 21. The outer tube 22 is provided with a balloon expanding lumen 220 formed between itself and the outer circumference 21b of the inner tube 21.

The outside diameter of the inner tube 21 is generally in the range of about 0.35 to 1 mm, preferably about 0.45 to 0.8 mm, and the inside diameter of the inner tube 21 is generally in the range of about 0.2 to 0.9 mm, preferably about 0.35 to 0.7 mm.

The outside diameter of the outer tube 22 is generally in the range of about 0.6 to 1.5 mm, preferably about 0.8 to 1.1 mm, and the inside diameter of the outer tube 22 is generally in the range of about 0.5 to 1.4 mm, preferably about 0.7 to 1 mm.

The material for forming the outer tube 22 is preferably a material which has a certain degree of flexibility. Examples of the material include thermoplastic resins such as polyolefines (e.g., polyethylene, polypropylene, ethylene-propylene copolymer, ethylene-vinyl acetate copolymer or the like, and, further, crosslinked or partly crosslinked products thereof), polyvinyl chloride, polyamides such as nylon 12 and nylon 11, polyamide elastomers, polyurethane, etc.; silicone rubbers, latex rubbers, etc. Among these materials, preferred are the thermoplastic resins, and more preferred are crosslinked or partly crosslinked polyolefins. Blends of these materials such as blend of polyamides and polyamide elastomers can also be used.

In the present invention, among the tubular members constituting the catheter with expandable member 1, the inner tube 21 has a inside surface 21a of its lumen composed of a specified composite material. The inner tube 21 and the composite material will be described in detail later.

The balloon 3 can be folded and expanded according to variations in the internal pressure, and comprises a barrel portion 30 which is expanded into a tubular shape (preferably, a hollow cylindrical shape) with a substantially constant diameter along its longitudinal direction by a fluid injected therein, a proximal end reduced diameter portion 32 connected to the barrel portion 30 through a gradually reduced diameter portion (taper portion) 31 gradually reduced in diameter toward the proximal side, and a distal end reduced diameter portion 34 connected to the barrel portion 30 through a gradually reduced diameter portion (taper portion) 33 gradually reduced in diameter toward the distal side. The barrel portion 30 in its expanded state may not necessarily be perfectly hollow cylindrical in shape, and may be polygonal-base columnar in shape. The balloon 3, in an unexpanded shape (not shown), can be folded onto the outer circumference of the inner tube 21.

The balloon 3 in its expanded state forms an expansion space 300 between itself and the outer circumference 21b of the inner tube 21. The proximal end portion side of the expansion space 300 is communicated with the balloon expanding lumen 220 of the outer tube 22, along the entire circumference thereof. Since the expanding lumen 220 has a comparatively large volume, injection of an expanding fluid into the balloon 3 (expansion space 300) is assured.

The distal end reduced diameter portion 34 is attached liquid-tightly to the outer circumference 21b of the inner tube 21, and the proximal end reduced diameter portion 32 to the distal end 221 of the outer tube 22, by use of an adhesive or by heat fusion or the like.

The method for joining the expandable member 3 to the tubular members (the inner tube 21 and the outer tube 22) includes a method of attaching the inner tube 21 for passing the guide wire therethrough and the expandable member 3 to each other by adhesion, and a method of attaching them by heat fusion. The heat fusion method is advantageous, since a reduction in diameter of the fused portion can be easily achieved by thermal working after heat fusion and flexibility of the fused portion can be easily obtained.

In the case where a stent (not shown) is mounted, the stent is mounted around the barrel portion 30 having a substantially same diameter along its longitudinal direction in the condition where the balloon 3 is folded, and then the stent is expanded by the expanding force of the balloon 3.

The size of the balloon 3 is not particularly limited. The outside diameter of the hollow cylindrical portion (barrel portion 30) in its expanded state is generally in the range of 1.5 to 6 mm, preferably 2 to 4 mm, and the length thereof is generally in the range of 10 to 50 mm, preferably 10 to 40 mm. The outside diameter of the distal end reduced diameter portion 34 is generally 0.5 to 1.5 mm, preferably 0.6 to 1.3 mm, and the length thereof is generally 1 to 5 mm, preferably 1 to 2.0 mm. In addition, the outside diameter of the proximal end reduced diameter portion 34 is generally 0.5 to 1.6 mm, preferably 0.7 to 1.5 mm, and the length thereof is generally 1 to 5 mm, preferably 2 to 4 mm. Besides, the entire length of the gradually reduced diameter portion 31 on the proximal end side is generally 1 to 10 mm, preferably 3 to 7 mm. The entire length of the gradually reduced diameter portion 33 on the distal end side is generally 1 to 10 mm, preferably 3 to 7 mm.

The material forming the balloon 3 is preferably a material which has a certain degree of flexibility. Examples of the material usable include thermoplastic resins such as polyolefins (e.g., polyethylene, polypropylene, ethylene-propylene copolymer, ethylene-vinyl acetate copolymer, crosslinked ethylene-vinyl acetate polymer or the like), polyvinyl chloride, polyamides such as nylon 12 and nylon 11, polyamide elastomers, polyurethane, polyesters (e.g., polyethylene terephthalate), polyarylene sulfides (e.g., polyphenylene sulfide), etc.; silicone rubbers, latex rubbers, etc. Among these materials, orientable materials are particularly preferred. The balloon 3 is preferably formed of a biaxially oriented material having high strength and expansion force. Laminates and blends of two or more of the above-mentioned materials may also be used.

In the catheter with expandable member 1 according to the present invention, the inside surface 21a of the inner tube 21 is composed of a specified composite material, namely, a composite material comprising nanocarbon dispersed in a polymer matrix. In an embodiment shown in Fig. 2, the inner tube 21 is composed of a single layer of the composite material.

As the polymer constituting the matrix of the composite material, those polymer materials which can generally be used for medical use can be widely used, whether they may be thermoplastic, thermosetting or thermo-crosslinking, as long as they have a certain degree of flexibility. Specific examples of the material usable include thermosetting resins or thermo-crosslinking resins polyolefins such as polyethylene, polypropylene, and ethylene-propylene copolymer; olefin-based copolymers such as ethylene-vinyl acetate copolymer, ethylene-vinyl acetate saponified product, and ethylene-vinyl alcohol copolymer, and polyolefin elastomers; vinyl-based polymers such as polyvinyl chloride (PVC), polyvinylidene chloride (PVDC), and polyvinylidene fluoride (PVDF); polyamides (PA) generically called nylon (trade name) such as nylon 6, nylon 11, nylon 12, nylon 46, nylon 60, polyhexamethylene adipamide (nylon 6,6), polyhexamethylene azelamide (nylon 6,9), polyhexamethylene sebacamide (nylon 6,10), polyhexamethylene dodecanamide (nylon 6,12), and nylon MXD6, and nylon-based copolymers containing at least one component of these; polyamide elastomers (PAE) (e.g., polyamide block copolymers comprising polyether-based soft segments (e.g. polyether-ester amide or polyether amide) and hard segments of polyamide); polyesters such as polyethylene terephthalate, and polybutylene terephthalate, and polyester elastomers containing these as hard segments; polyimides, polystyrene, SEBS resin, polyurethane, polyurethane elastomers, silicone rubbers, latex rubbers and the like.

These materials may be used either singly or in combination of two or more of them. Polymer alloys, for example, those containing a polymer containing one of the above-mentioned materials may also be utilized. Among these materials, thermoplastic polymers which can be formed into tubes by extrusion are preferably used.

Particularly, the polymer constituting the matrix of the composite material is preferably composed of a material which is compatible with the material forming the balloon 3 mentioned above, since the heat fusion between itself and the balloon 3 can be performed favorably.

Examples of the combination of such mutually compatible materials include combinations of a polyolefin-based balloon material with a polyolefin-based matrix polymer, combinations of a polyamide-based or a polyamide elastomer-based balloon material with a polyamide-based or a polyamide elastomer-based matrix polymer, combinations of a polyester elastomer-based balloon material with a polyester elastomer-based matrix polymer, and combinations of a PET or PBT balloon material with a polyester elastomer-based matrix polymer.

The nanocarbon used in the present invention is a generic name for three-dimensional hollow structures whose profiles are composed of carbon net surfaces and whose sizes are nanometer (nm) order size. The nanometer order size means that the diameter (D) of the nanocarbon is up to about 2000 nm at maximum. In the case of fiber-formed structures or the like, the fiber length may be several tens of micrometers (µm) or more, as long as the diameter is nanometer order size.

As the three-dimensional structures, there have been known hollow granular structures or fiber-formed structures in various shapes such as roughly (circular) tubular shapes, cage-like shapes, roughly spherical shapes. Examples include fullerenes in nearly spherical granular form. Fullerences include not only those which have hitherto been well known, such as C₆₀ (spherical structure having 60 carbon atoms and a diameter of about 0.7 nm) and C₇₀, but also those which have 70 or more carbon atoms, those having a multi-wall structure, such as dimer and trimer of C₆₀, and nearly spherical stable small fullerences having less than 60 carbon atoms.

Examples of the fiber-formed structures include carbon nanotube which is a single roughly hollow cylindrical structure in the shape formed by rounding a single layer sheet of graphite, in which the carbon net surfaces are roughly parallel to the fiber axis. Specific examples include single-wall carbon nanotubes (SWNT) having a diameter (fiber diameter) of about 1 to 10 nm, multi-wall carbon nanotubes (MWNT) in a nesting or telescopic structure comprising two or more (circular) tubular structures overlapping each other (maximum diameter: several to several tens of nanometers), and vapor grown carbon nanotube (VGCF) having a large diameter of not less than 100 nm. In addition, carbon nanofibers (CNF) having a diameter of several tens to several hundreds of nanometers include not only those in which the carbon net surfaces are roughly parallel to the fiber axis but also those in which the carbon net surfaces are inclined against or perpendicular to the fiber axis. Examples of such carbon nanofibers (CNF) include carbon nanohorns with a diameter of about 1 to several nm, and carbon nanofibers in the shapes usually called cup stack type.

In the present invention, the nanocarbon is not particularly limited to the above-mentioned examples. Among the exemplary nanocarbons, preferred are the fiber-formed structures, in view of a sufficient reinforcement effect. The fiber length (L) is preferably not less than 0.1 µm, and the aspect ratio (L/D) is desirably not less than 5, preferably not less than 10, and more preferably not less than 150.

On the other hand, in view of sufficient dispersibility of the nanocarbon into the matrix polymer, the maximum fiber length is preferably up to about 1000 µ m, and the aspect ratio is up to about 10000, preferably not more than about 1000. Incidentally, the aspect ratio is in terms of average for all nanocarbons, and, therefore, it is not necessary that all the nanocarbons used should individually satisfy the above-mentioned range of aspect ratio. Therefore, the nanocarbons are not limited to the fiber-formed structures such as nanotubes and nanofibers, and may include nearly spherical particles such as fullerenes.

The method for producing the nanocarbon as above-mentioned is not particularly limited. Examples of the method usable include the arc method, the laser ablation method and the like which have been known, and also include the production methods proposed for mass production or for obtaining a desired structure. In the present invention, the existing products of nanocarbon can be used. For example, nanocarbons produced based on the mass synthesis technology progressively researched in the Carbon-based High-functional Material Technology Project of the Japanese Ministry of Economy, Trade and Industry are available, and other commercialized products can be used.

The above-mentioned nanocarbon may have been surface treated for enhancing wettability, adhesiveness or the like between the nanocarbon and the polymer. For example, the surface of the nanocarbon may have been subjected to a degreasing treatment or a washing treatment, or may have been subjected to an activation treatment such as a UV irradiation treatment, a corona discharge treatment, a plasma treatment, a flame treatment, an ion injection treatment, etc. For easy dispersion and mixing of the nanocarbon into the polymer, the nanocarbon having been subjected to the above-mentioned surface treatment may further be treated with a coupling agent based on silane, titanium, aluminum or the like.

In preparation of the composite material from the above-mentioned polymer and nanocarbon, the ratio between the amounts of the polymer and the nanocarbon used is appropriately set according to the final size of the tube formed from the composite material, the material of the matrix polymer and/or the kind of nanocarbon and the like. Thus, the ratio varies depending on various factors, and is not particularly limited. To obtain a composite material of the structure comprising the nanocarbon dispersed in the polymer matrix, it is preferable that the amount of nanocarbon is not more than 40% by weight based on the total amount of polymer and nanocarbon. When the amount of nanocarbon increased in excess of 40% by weight, it becomes difficult to hold the polymer matrix, the rupture strength of the composite material tends to be lowered, and it becomes difficult to secure the desired low-friction property. The amount of nanocarbon based on the total amount of polymer and nanocarbon is preferably not more than 20% by weight, and more preferably not more than 10% by weight. On the other hand, for securing the reinforcement effect arising from the nanocarbon and for obtaining the low-friction property, the amount of nanocarbon is preferably not less than 1% by weight, more preferably not less than 3% by weight.

In the present invention, the preparation of the composite material composed of the above-mentioned polymer and nanocarbon and the tube forming may be conducted as a continuous process. Alternatively, a composition in the form of pellets or powder prepared by compounding the polymer and the nanocarbon in advance may be served to the tube forming.

In preparation of the composite material, a known compounding technique can be appropriately adopted if a composition containing the nanocarbon dispersed substantially uniformly in the polymer matrix can be obtained. Examples of the compounding technique adoptable include the methods of kneading the polymer and the nanocarbon by use of a single-screw or twin-screw kneader, rubber rolls, a stone mortar type kneader or the like. The examples also include the method of adding the nanocarbon to a solution of the polymer in an appropriate solvent, and mixing the resultant mixture.

Further, favorable examples of the method for dispersing fine short fibers such as the nanocarbon used in the present invention into a polymer include the method in which the synthesis of the polymer and the mixing of the nanocarbon are simultaneously conducted by in-situ polymerization. Specifically, the nanocarbon may be dispersed in a polymerizable raw material before polymerization reaction of the polymer for constituting the matrix of the composite material or in a low-viscosity polymer before completion of polymerization (or a precursor thereof: polymerizable monomer, principal agent/curing agent of thermoplastic resin), whereby dispersibility can be largely improved. This method is particularly effective in the case of a polymer such that sufficient dispersibility of nanocarbon cannot be obtained by only fusion kneading.

In addition, for enhancing the dispersibility of the nanocarbon into the polymer matrix, it is preferable to apply the ultrasonic shaking method. Namely, when ultrasonic vibration is applied to the nanocarbon-polymer mixture for a predetermined period of time, the polymer is made to permeate between the nanocarbon particles while disentangling the partly coagulated nanocarbon particles and spacing them wider, whereby the nanocarbon can be dispersed into the polymer matrix.

Though the ultrasonic shaking method can be applied to the polymer in a molten state, it is effective when applied to the polymer in a solution state. The mixing between the polymer solution and the nanocarbon can be conducted by only the ultrasonic shaking method or by using the method together with stirring vanes, whereby the polymer solution is made to permeate between the nanocarbon particles, and the nanocarbon can be dispersed in the polymer.

The polymer in the solution state may be a polymer in the process of production by solution polymerization. Thus, there can be favorably used the method of adding the nanocarbon to a polymerizable raw material solution before the start of polymerization or to the polymerization solution during or after polymerization, and applying ultrasonic vibration to the resultant admixture.

By applying the above-mentioned method, a composite material containing the nanocarbon dispersed at random in the polymer matrix can be obtained, and such dispersion enhances the interfacial adhesive force between the nanocarbon and the polymer.

The inside surface 21a of the inner tube 21 is formed of the composite material, and, due to the presence of the nanocarbon in the composite material forming the inside surface 21a, the inside surface 21a is provided with minute recesses and projections. The presence of the minute recesses and the projections reduces the area of contact between the inside surface 21a of a guide wire lumen 210 and the guide wire (not shown), thereby reducing the frictional resistance between the inside surface 21a of the guide wire lumen 210 and the guide wire. As a result, as compared with the case of the inner tube 21 formed by polymers only, the frictional resistance between the inside surface 21a of the guide wire lumen 210 and guide wire is reduced, and the slidability of the guide wire is enhanced. Therefore, it is possible to provide a catheter in which the guide wire would not be stuck or locked on the surface 21a of the guide wire lumen 210 and which is excellent in operation property and safety.

According to one embodiment of the present invention, the recesses and projections are formed by a configuration in which the profiles of the nanocarbon are embossed at the inside surface 21a of the inner tube 21 to a certain extent, though the nanocarbon is covered with the matrix polymer and is not exposed on the inside surface 21a. On the other hand, according to another embodiment of the present invention, the recesses and projections are formed by a configuration in which the nanocarbon is exposed on the inside surface 21a of the inner tube 21.

The nanocarbon may be present over the entire region of the inner tube 21, as in the case of the inner tube 21 of the single-layer structure shown in Fig. 2, or may be present only at the inside surface 21a of the inner tube 21.

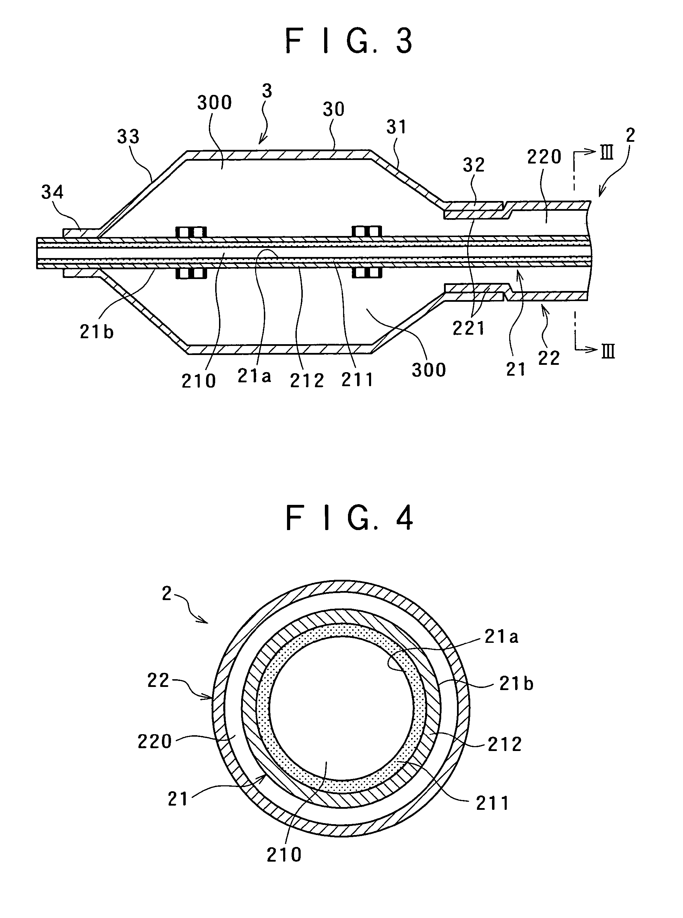

Fig. 3 is an enlarged sectional view of a portion near the distal end of the catheter with expandable member showing another embodiment of the present invention, and Fig. 4 is a sectional view along line III-III of Fig. 3.

In this embodiment, the inner tube 21 has a two-layer (multi-layer) structure composed of an outer layer 212 formed of a polymer not containing nanocarbon and an inner layer 211 formed of a nanocarbon-containing composite material.

As an alternative to, while the inner layer 211 is formed of the above-mentioned composite material, the outer layer 212 may be formed of the polymer in which the nanocarbon is blended. As the matrix polymer for forming the outer layer 212, the same materials as that in the inner layer 211 can be used. It is preferable that the material used for forming the outer layer 212 is the same material as the matrix polymer of the inner layer 211 or a material compatible with the matrix polymer of the inner layer 211. Besides, when a material more flexible than the matrix polymer of the inner layer 211 is used for the matrix polymer of the outer layer 212, it is possible to provide the inner tube 21 as a whole with sufficient flexibility.

Where nanocarbon is blended in the outer layer 212, the adhesiveness between the outer layer 212 and the inner layer 211 can be further enhanced, and it is possible to obtain the inner tube 21 in which the possibility of interlayer exfoliation between the inner layer 211 and the outer layer 212 is further reduced. The amount of nanocarbon blended in the polymer of the outer layer 212 is not particularly limited. The nanocarbon content in the outer layer 212 is preferably less than the content of the nanocarbon in the inner layer 211, for providing the inner tube 21 as a whole with sufficient flexibility and for not spoiling the compatibility between the balloon 3 and the inner tube 21 (i.e. the outer layer 212) in the case of fusing them to each other.

In the two-layer structure, the matrix polymer used for forming the inner layer 211 is preferably has a lower-friction property as compared with the polymer of the outer layer 212. In addition, the matrix polymer used for forming the inner layer 211 is preferably has a lower dynamic friction property as compared with the polymer of the outer layer 212. That means the coefficient of dynamic friction of the matrix polymer used for forming the inner layer 211 is lower than that of the matrix polymer used for forming the outer layer 212. As a result of this, in cooperation with the blending of the nanocarbon, it is possible to further reduce the frictional resistance between the inside surface 21a of the guide wire lumen 210 and the guide wire, and to further enhance the slidability (operation property) of the guide wire.

The content of the nanocarbon in the inner layer 211 can be set comparatively high, without spoiling the flexibility of the inner tube 21 as a whole, as compared with the single-layer inner tube 21 of Fig.2. This makes it possible to further reduce the frictional resistance between the inside surface 21a of the guide wire lumen 210 and the guide wire. Therefore, it is possible to provide an appliance in which the shaft main body portion 2 (inner tube 21) has good flexibility, which can sufficiently follow up to a bent portion of a blood vessel, which is excellent in the slidability of the guide wire, and which is excellent also in operation property and safety.

From the foregoing, examples of preferable combination of the matrix polymer of the outer layer 212 with the matrix polymer of the inner layer 211 include the followings.
(1) The outer layer 212 is formed of a polyamide elastomer, while the inner layer 211 is formed of a polyamide (e.g., a nylon material such as polyhexamethylene adipamide (nylon 6,6), polyhexamethylene azelamide (nylon 6,9), polyhexamethylene sebacamide (nylon 6,10), polyhexamethylene dodecanamide (nylon 6,12), nylon 6, nylon 11 and nylon 12); a polyamide elastomer lower in soft segment content (namely, higher in hardness) than the polyamide elastomer material of the outer layer which has a higher hardness and a lower-friction property or a lower dynamic friction property as compared with the polyamide elastomer of the outer layer 212.
(2) The outer layer 212 is formed of a polyester elastomer, while the inner layer 211 is formed of a polyester elastomer lower in soft segment content (namely, higher in hardness) than the polyester elastomer of the outer layer 212.
(3) The inner layer 211 is formed of a polyolefin such as polyethylene and polypropylene, while the outer layer 212 is formed of a polyolefin-based elastomer.

On the other hand, from the viewpoint of making it possible to provide an excellent low-friction property, the matrix polymer of the inner layer 211 is preferably a polyolefin such as polyethylene and polypropylene (more preferably, polyethylene, and particularly preferably a high-density polyethylene). For enabling heat fusion to the polyamide-based or polyester-based balloon, the matrix polymer of the outer layer 212 is preferably a polyamide, polyamide elastomer or polyester elastomer which is incompatible with the polyolefin of the inner layer 211.

In this case, for making sufficient the adhesiveness (close contact property) between the two layers, it is preferable that an adhesive polymer having adhesiveness to both of the matrix polymer of the inner layer 211 and the matrix polymer of the outer layer 212 is contained in at least one of the inner layer 211 and the outer layer 212 or that an intermediate adhesive layer (not shown) containing the adhesive polymer is provided between the inner layer 211 and the outer layer 212.

Preferable examples of the adhesive polymer include modified polyolefins obtained by co-polymerization of the polyolefin, such as polyethylene, polypropylene and ethylene-vinyl acetate copolymer, with a monomer having a functional group of, for example, an unsaturated carboxylic acid such as maleic acid, fumaric acid, cinnamic acid, crotonic acid, and linoleic acid. Other examples of the adhesive polymer include acid-functionalized ethyl vinyl acetate resins, acid-functionalized ethylene acrylate polymers, anhydrous-functionalized ethyl vinyl acetate copolymers, acid- and acrylate-functionalized ethyl vinyl acetate resins, anhydrous-functionalized ethyl vinyl acetate copolymers, and anhydrous-functionalized ethyl vinyl acetate resins.

Further, the outer layer 212 is preferably composed of a material compatible with the above-mentioned material forming the balloon 3, in view of good heat fusion thereof to the balloon 3. Specifically, the matrix polymer of the outer layer 212 is preferably composed of a material compatible with the material forming the balloon 3. Examples of the combination of compatible materials include combinations of a polyolefin-based balloon material with a polyolefin-based matrix polymer, combinations of a polyamide-based or a polyamide elastomer-based balloon material with a polyamide-based or a polyamide elastomer-based matrix polymer, combinations of a polyester elastomer-based balloon material with a polyester elastomer-based matrix polymer, and a PET or PBT balloon material with a polyester elastomer-based matrix polymer.

In the embodiment of the multi-layer structure, the amounts of the matrix polymer and the nanocarbon blended in the inner layer 211 can be appropriately set according to the size of the catheter tube, the material of the matrix polyer and the like, and are not particularly limited. If the amount of the nanocarbon blended is too small, a sufficient low-friction property could not be obtained. Therefore, the amount of the nanocarbon based on the total weight of matrix polymer and nanocarbon is preferably not less than 1% by weight, more preferably not less than 5% by weight. With the amount of the nanocarbon set to be not more than 20% by weight, the nanocarbon and the matrix polymer can be mixed favorably, whereby it is possible to prevent mechanical strength of the inner tube 21 (inner layer 211) from being lowered due to unsatisfactory mixing.

Besides, in the case of blending nanocarbon also in the outer layer 212, the amounts of the matrix carbon and the nanocarbon blended in the outer layer 212 can be appropriately set according to the size of the inner tube 21, the material of the matrix polymer and the like, and are not particularly limited. The amount of the nanocarbon based on the total weight of matrix polymer and nanocarbon is preferably not more than 10% by weight, more preferably not more than 5% by weight.

Incidentally, while the inner tube 21 has the double-layer structure in the embodiment shown in Fig. 3, the present invention is not limited to this structure. The inner tube may be provided with three or more layers, by providing one or more layers between the inner layer 211 and the outer layer 212. In this case, the layer (intermediate layer) disposed between the inner layer 211 and the outer layer 212 can be formed of a material obtained by mixing nanocarbon in the matrix forming the layer (intermediate layer). Particularly, with the nanocarbon blended in the intermediate layer in an amount which is less than the amount of nanocarbon blended in the inner layer 211 and is more than the amount of nanocarbon in the outer layer 212, the intermediate layer is suitable as an intermediate joint layer for joining the inner layer 211 and the outer layer 212 to each other further strongly.

It should be noted here that when the inner tube 21 is composed only of two layers, the extrusion process can be easily conducted with less equipment and production cost is lowered, as compared with the case where the inner tube 21 is composed of three or more layers.

In present invention, the arithmetical mean roughness of the inside surface 21a of the inner tube 21 is preferably not less than 0.1 am in terms of efficiently reducing the area of contact between the inside surface 21a of the guide wire lumen 210 and the guide wire so as to efficiently reduce the frictional resistance between the inside surface 21a of the guide wire lumen 210 and the guide wire. As a result, the slidability and operation property of the guide wire in the guide wire lumen is enhanced.

The surface roughness of the inside surface 21a of the inner tube 21 as above-described can be obtained in terms of an arithmetical mean roughness, as follows.

For a inside surface 21a of the inner tube 21 cut in the longitudinal direction, the arithmetical mean roughness calculated from a section profile of the inner tube 21 can be measured by use of a laser microscope (VK-8500, produced by KEYENCE) based on JIS B0601. In this case, taking into account the noises generated in the measurement by the laser microscope in the vicinity of the cut section of the inner tube 21, it is desirable to use in measurement a 45-µm long portion in a central portion of the inner tube 21 sufficiently spaced from the cut section.

### [Examples]

Now, the present invention will be described more in detail below referring to Examples, which are not limitative of the invention.

### [Example 1]

### <Preparation of Composite Material Pellets>

Compounding of 80 parts by weight of a polyamide elastomer resin (a polyether-ester block amide containing polyether soft segments and polyamide hard segments joined to each other through ester linkage; having a Shore D hardness of 54) and 20 parts by weight of carbon nanofibers (average outside diameter: about 150 nm; length: about 10 to 20 µm; produced by Showa Denko K.K.) was conducted by use of a twin-screw kneader, followed by extrusion and cutting, to obtain pellets of a composite material having a carbon nanofiber content of 20% by weight.

### <Production of Inner Tube>

Mixing of 30 parts by weight of the pellets of the carbon nanofiber-containing composite material and 70 parts by weight of pellets of nylon 12 (Shore D hardness: 72) was conducted by the ordinary method, and the resultant mixture was extruded, to produce a single-layer tube inner tube having an outside diameter of 0.56 mm and an inside diameter of 0.43 mm.

The amount of the carbon nanofiber (based on the total weight of nylon 12, the polyamide elastomer and the carbon nanofibers) in the inner tube was 6% by weight.

### [Example 2]

Compounding of 90 parts by weight of nylon 12 (Shore D hardness: 72) and 10 parts by weight of carbon nanofibers (average outside diameter: about 150 nm; length: about 10 to 20 µm; produced by Showa Denko K.K.) was conducted by use of a twin-screw kneader, followed by extrusion and cutting, to obtain pellets of a composite material having a carbon nanofiber content of 10% by weight.

With the carbon nanofiber-containing composite material used as a material for forming an inner layer and with a polyamide elastomer resin (a polyether-ester block amide containing polyether soft segments and polyamide hard segments joined to each other by ester linkage; having a Shore D hardness of 54) used as a material for forming an outer layer, copper wire covering was conducted by use of a multi-layer extruding machine, to form on a copper wire an inner tube of a two-layer tube structure having an outside diameter of 0.56 mm and an inside diameter of 0.43 mm (the outer layer having a material thickness of about 0.06 mm, and the inner layer having a material thickness of about 0.07 mm).

Incidentally, the area ratio between the outer layer forming material and the inner layer forming material supplied to the multi-layer extruding machine was 1:1. The extrusion was conducted at a temperature of 200 °C, and the copper wire had an outside diameter of 0.43 mm.

### [Comparative Example 1]

An inner tube of a single-layer tube structure was produced in the same manner as in Example 1, except that the carbon nanofibers were not blended and only nylon 12 and the polyamide elastomer were used.

### [Comparative Example 2]

An inner tube of a single-layer tube structure was produced in the same manner as in Example 2, except that the carbon nanofibers were not blended and only the matrix resin was used.

### <Three-Point Bending Test>

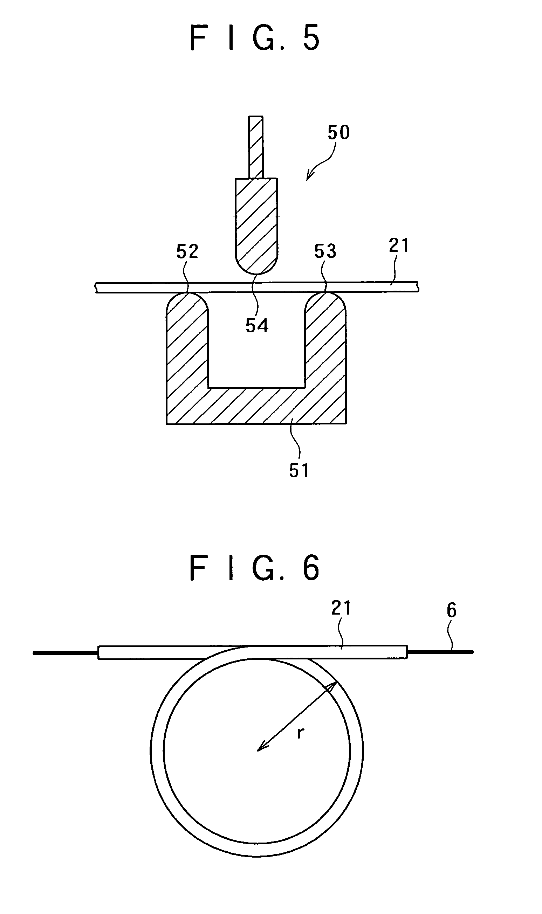

For the inner tubes produced in Examples 1 and 2 and Comparative Examples 1 and 2, a three-point bending test was conducted by use of a jig 50 shown in Fig. 5, for examining the bending strength which serves as an index of flexibility.

First, the inner tube 21 was placed on edges 52 and 53 (the distance between the edges 52 and 53 was 2.5 cm) of a base 51, whereby a portion of the inner tube 21 located between the edges 52 and 53 was pressed downwards by 2 mm by use of an edge 54, and the maximum load on the edge 54 was measured. The pressing-in speed of the edge 54 was 5 mm/min, the length of the inner tube 21 used in the measurement was 250 mm, and the measurement was conducted at room temperature (20 °C). The results are shown in Table 1 below.

### <Guide Wire Slidability Test - 1>

A guide wire (produced by TERUMO Corporation; trade name: Runthrough; outside diameter: 0.36 mm) 6 was passed through each of the inner tubes 21 obtained in Examples 1 and 2 and Comparative Examples 1 and 2, and each wire was curved so as to form a circle with an outside diameter of 50 mm (r: 25 mm). In this condition, the guide wire was slid 30 times at a test speed of 100 mm/min and a stroke of 20 mm, and the maximum resistance during the sliding was measured (measurement temperature: 20 °C). The results are shown in Table 1 below.

As for the tact, the inner tube produced in Example 2 was high in the feeling of slide (feeling of rustle), while the inner tube produced in Example 1 showed a little heavy feeling (feeling of tardiness) at the time of sliding the guide wire.

**Table 1**

| | Inner tube | | Bending strength gf (N equivalent) (n=3) | Sliding resistance gf (N equivalent) (n=5) |
|---|---|---|---|---|
| | Configuration | Carbon Nanofiber content in inside surface (wt.%) | | |
| Example 1 | Single layer | 6 | 2.73 (0.027) | 5.60 (0.055) |
| Example 2 | Double layer | 10 | 2.63 (0.026) | 5.56 (0.054) |
| Comparative Example.1 | Single layer | 0 | 2.57 (0.025) | 8.34 (0.082) |
| Comparative Example. 2 | Double layer | 0 | 2.30 (0.023) | 7.40 (0.073) |

From the results shown in Table 1, it is seen that Example 1 gave a bending strength of about 106% and a sliding resistance of about 67% on the condition that the bending strength and sliding resistance in Comparative Example 1 is 100% respectively. It is also seen that Example 2 gave a bending strength of about 114% and a sliding resistance of about 75% on the condition that the bending strength and sliding resistance in Comparative Example 2 be 100% respectively.

From these results, it has been confirmed that blending of carbon nanofibers increases bending strength and markedly reduces the sliding resistance of guide wire.

Besides, comparison of Example 1 with Example 2 has verified that, though the sliding resistances in the Examples are substantially equal, Example 2 gave a lower bending strength, which indicate flexibleness.

### [Example 3]

Compounding of 95 parts by weight of nylon 12 (Shore D hardness: 72) and 5 parts by weight of carbon nanofibers (average outside diameter: about 150 nm; length: about 10 to 20 µm; produced by Showa Denko K.K.) was conducted by use of a twin-screw kneader, followed by extrusion and cutting, to obtain pellets of a composite material containing 5% by weight of the carbon nanofibers.

The carbon nanofiber-containing composite material was extruded by the ordinary method, to produce an inner tube of a single-layer tube structure having an outside diameter of 0.56 mm and an inside diameter of 0.43 mm.

### [Example 4]

An inner tube of the single-layer structure was produced in the same manner as in Example 3, except that the carbon nanofiber content was set to 10% by weight.

### [Comparative Example 3]

An inner tube of the single-layer tube structure was produced in the same manner as in Examples 3 and 4, except that the carbon nanofibers were not blended and only nylon 12 was used.

### <Guide Wire Slidability Test - 2>

For the inner tubes obtained in Examples 3 and 4 and Comparative Example 3, slidability of a guide wire in blood was tested, by the following test method.

A guide wire 6 was passed through each of the inner tubes obtained in Examples 3 and 4 and Comparative Example 3, in the same manner as in Guide Wire Slidability Test - 1, except that the distal end of the inner tube was sealed with a three-way stopcock and the inner tube was filled with rabbit blood. In the condition where the inner tube with the guide wire was curved, the guide wire was slid 30 times at a test speed of 100 mm/min and a stroke of 20 mm, and the maximum resistance during the sliding was measured (measurement temperature: 20 °C). The results are shown in Table 2 below.

**Table 2**

| | Carbon nanofiber content in inside portion of inner tube (wt.%) | Sliding resistance Gf (N equivalent) (n=2) |
|---|---|---|
| Example 3 | 5 | 16 (0.157) |
| Example 4 | 10 | 13 (0.127) |
| Comp.Ex.3 | 0 | 18 (0.176) |

From the results shown in Table 2, it has been confirmed that the inner tube containing 10% by weight of the carbon nanofibers was lower in sliding resistance than the inner tube containing 5% by weight of the carbon nanofibers.

### <Production of Catheter with Expandable Member>

A distal end portion of a φ3.5/20 mm balloon (hollow cylindrical barrel portion having an outside diameter of 3.5 mm and a length of 20 mm; material: polyamide elastomer resin (a blend of a polyether-ester block amide comprising polyether soft segments and polyamide hard segments joined to each other through ester linkage, having a Shore D hardness of 54, and another similar polyether-ester block amide having a Shore D hardness of 62)) was heat fused to the distal end of each of the inner tubes obtained in the above Examples, and a proximal end portion of the balloon was heat fused to an outer tube (material: a blend of nylon 12 and the polyamide elastomer resin used in Example 1). The flexibility of each of the heat-fused portions was evaluated, by touch, to be sufficiently soft.

The present invention is not limited to the details of the above described preferred embodiments. The scope of the invention is defined by the appended claims and all changes and modifications as fall within the equivalence of the scope of the claims are therefore to be embraced by the invention.

## Claims

1. A catheter (1) with expandable member (3), comprising a guide wire guiding tubular member (21) having an inside surface (21a) of its lumen (220) composed of a composite material containing nanocarbon dispersed in a matrix polymer, and an expandable member (3) disposed around the outer circumference of a portion in the vicinity of a distal end portion of said tubular member (21), a distal end portion of said expandable member (3) being attached to the outer circumference of the portion in the vicinity of said distal end portion of said tubular member (21), **characterized in that**
said lumen surface of said tubular member (21) has a rough surface having minute recesses and projections.

2. The catheter (1) with expandable member (3) according to claim 1, wherein said matrix polymer is compatible with the material constituting said expandable member (3).

3. The catheter (1) with expandable member (3) according to claim 1 or 2, wherein the attachment of said expandable member (3) to said tubular member (21) is conducted by heat fusing.

4. The catheter (1) with expandable member (3) according to claim 1 or 2, wherein said tubular member (21) has an outer layer (212) and said outer layer (212) comprises a matrix polymer compatible with said matrix polymer of said inside surface (21 a) and with the material constituting said expandable member (3).

5. The catheter (1) with expandable member (3) according to any of claims 1 to 3, wherein said tubular member (21) has an outer layer (212) which comprises a thermoplastic elastomer compatible with said matrix polymer of said inside surface (21 a) and which is higher in frictional property than said inside surface (21a).

6. The catheter (1) with expandable member (3) according to claim 5, wherein said thermoplastic elastomer forming said outer layer (212) is a polyamide elastomer, and said matrix polymer forming said inside (21a) surface is a polyamide which is higher in hardness and lower in frictional property than said polyamide elastomer.

7. The catheter (1) with expandable member (3) according to claim 1, wherein the arithmetical mean roughness of said rough surface calculated from a profile curve of a longitudinal section of said tubular member (21) is not less than 0.1 mu m.

8. The catheter (1) with expandable member (3) according to any of claims 1 to 7, further comprising a stent mounted onto the outside surface of said expandable member (3).

## Patentansprüche

1. Katheter (1) mit einem ausdehnbaren Element (3), der ein einen Führungsdraht führendes röhrenförmiges Element (21), das eine Innenfläche (21a) seines Lumens (220) hat, die aus einem Verbundwerkstoff zusammengesetzt ist, das Nanokohlenstoff, feinstverteilt in einem Matrixpolymer, enthält, und ein ausdehnbares Element (3), das um den Außenumfang eines Abschnitts in der Nähe eines distalen Endabschnitts des röhrenförmigen Elements (21) angeordnet ist, umfasst, wobei ein distaler Endabschnitt des ausdehnbaren Elements (3) an dem Außenumfang des Abschnitts in der Nähe des distalen Endabschnitts des röhrenförmigen Elements (21) befestigt ist, **dadurch gekennzeichnet, dass**
die Lumenfläche des röhrenförmigen Elements (21) eine raue Oberfläche hat, die winzige Aussparungen und Vorsprünge hat.

2. Katheter (1) mit einem ausdehnbaren Element (3) nach Anspruch 1, wobei das Matrixpolymer mit dem Material, welches das ausdehnbare Element (3) ausmacht, verträglich ist.

3. Katheter (1) mit einem ausdehnbaren Element (3) nach Anspruch 1 oder 2, wobei die Befestigung des ausdehnbaren Elements (3) an dem röhrenförmigen Element (21) durch Warmverschweißen ausgeführt ist.

4. Katheter (1) mit einem ausdehnbaren Element (3) nach Anspruch 1 oder 2, wobei das röhrenförmige Element (21) eine äußere Lage (212) hat und die äußere Lage (212) ein Matrixpolymer umfasst, das mit dem Matrixpolymer der Innenfläche (21a) und mit dem Material, welches das ausdehnbare Element (3) ausmacht, verträglich ist.

5. Katheter (1) mit einem ausdehnbaren Element (3) nach einem der Ansprüche 1 bis 3, wobei das röhrenförmige Element (21) eine äußere Lage (212) hat, die ein thermoplastisches Elastomer umfasst, das mit dem Matrixpolymer der Innenfläche (21a) und verträglich ist und das in der Reibungseigenschaft höher ist als die Innenfläche (21a).

6. Katheter (1) mit einem ausdehnbaren Element (3) nach Anspruch 5, wobei das thermoplastische Elastomer, das die äußere Lage (212) bildet, ein Polyamid-Elastomer ist und das Matrixpolymer, das die Innenfläche (21a) bildet ein Polyamid ist, das in der Härte höher und in der Reibungseigenschaft niedriger ist als das Polyamid-Elastomer.

7. Katheter (1) mit einem ausdehnbaren Element (3) nach Anspruch 1, wobei die arithmetisch gemittelte Rauigkeit der rauen Oberfläche, berechnet aus einer Profilkurve eines Längsschnitts des röhrenförmigen Elements (21), nicht geringer als 0,1 µm ist.

8. Katheter (1) mit einem ausdehnbaren Element (3) nach einem der Ansprüche 1 bis 7, der ferner einen Stent umfasst, der an der Außenfläche des ausdehnbaren Elements (3) angebracht ist.

## Revendications

1. Cathéter (1) comportant un élément extensible (3), comprenant un élément tubulaire de guidage de fil guide (21) dont la lumière (220) a une surface intérieure (21 a) qui est faite d'un matériau composite contenant un nanocarbone dispersé dans un polymère formant matrice, et un élément extensible (3) placé autour de la circonférence extérieure d'une partie située au voisinage d'une partie d'extrémité distale dudit élément tubulaire (21), une partie d'extrémité distale dudit élément extensible (3) étant fixée à la circonférence extérieure de la partie située au voisinage de ladite partie d'extrémité distale dudit élément tubulaire (21), **caractérisé en ce que**
ladite surface de lumière dudit élément tubulaire (21) a une surface rugueuse comportant de minuscules creux et saillies.

2. Cathéter (1) comportant un élément extensible (3) selon la revendication 1, dans lequel ledit polymère formant matrice est compatible avec le matériau qui constitue ledit élément extensible (3).

3. Cathéter (1) comportant un élément extensible (3) selon la revendication 1 ou 2, dans lequel la fixation dudit élément extensible (3) sur ledit élément tubulaire (21) est réalisée par thermosoudage.

4. Cathéter (1) comportant un élément extensible (3) selon la revendication 1 ou 2, dans lequel ledit élément tubulaire (21) a une couche extérieure (212) et ladite couche extérieure (212) comprend un polymère formant matrice compatible avec ledit polymère formant matrice de ladite surface intérieure (21 a) et avec le matériau qui constitue ledit élément extensible (3).

5. Cathéter (1) comportant un élément extensible (3) selon l'une quelconque des revendications 1 à 3, dans lequel ledit élément tubulaire (21) a une couche extérieure (212) qui comprend un élastomère thermoplastique compatible avec ledit polymère formant matrice de ladite surface intérieure (21 a) et qui a une propriété de frottement supérieure à celle de ladite surface intérieure (21 a).

6. Cathéter (1) comportant un élément extensible (3) selon la revendication 5, dans lequel ledit élastomère thermoplastique qui forme ladite couche extérieure (212) est un élastomère à base de polyamide, et ledit polymère formant matrice qui forme ladite surface intérieure (21a) est un polyamide qui a une dureté plus grande et une propriété de frottement plus petite que ledit élastomère à base de polyamide.

7. Cathéter (1) comportant un élément extensible (3) selon la revendication 1, dans lequel la rugosité moyenne arithmétique de ladite surface rugueuse calculée à partir d'une courbe de profil d'une section longitudinale dudit élément tubulaire (21) est supérieure ou égale à 0,1 µm.

8. Cathéter (1) comportant un élément extensible (3) selon l'une quelconque des revendications 1 à 7, comprenant en outre une endoprothèse montée sur la surface extérieure dudit élément extensible (3).
